# EUROPEAN PATENT APPLICATION

(11) **EP 0 744 182 A2**
(43) Date of publication of application: **27.11.1996**
(21) Application number: 96107910.0
(22) Date of filing: 17.05.1996
(51) Int. Cl.: A61M 5/142

(54) **Self-administration device for liquid drugs**

(30) Priority: 24.05.1995 JP 124855/95
(71) Applicant: NISSHO CORPORATION, Osaka-shi, Osaka-fu (JP)
(72) Inventor: Hiejima, Katsuhiro, Ohtsu-shi, Shiga-ken (JP); Mori, Takeshi, Ibaraki-shi, Osaka-fu (JP); Nishiyama, Kazuhiko, Toyono-gun, Osaka-fu (JP)
(74) Representative: VOSSIUS & PARTNER

(57) **Abstract**

Self-administration device for liquid drugs includes a casing (11) having an open end and a closed end provided with inlet and outlet ports (12, 13) for a liquid drug, a deformable reservoir (15) arranged in the casing and capable of being restored to the original shape, and a pushing means (14) for depressing the reservoir. By operating the pushing means, it is moved along an inner or outer wall of the casing, brought into contact with the reservoir, and then presses the reservoir to deliver the liquid drug.

## Description

The present invention relates to a self-administration device for liquid drug (hereinafter referred to as "self-administration device") and, more particularly, to a device for injecting a liquid drug into the body of a patient to ease a pain such as a postoperative pain, cancerous pain or the like by himself or herself in combination with or without a system for continuously injecting a very small amount of a liquid drug such as analgesic or anticancer drug.

In the recent anesthetic field, the control of pains such as postoperative pains, cancerous pains and the like has been carried out by epidural catheterization in which a very small amount of a liquid drug such as an analgesic is continuously administered to a patient. However, symptoms varies with the patients and the patient occasionally complains of a sudden pain even when performing continuous microdose administration of analgesics. In order to cope with such a critical moment, there have been developed a device used for administering a dose of an analgesic to a patient by oneself. One example of such a device is a patient-controlled analgesic delivery system disclosed in international publication PCT/US86/01597 which corresponds to Japanese national publication 63-501195.

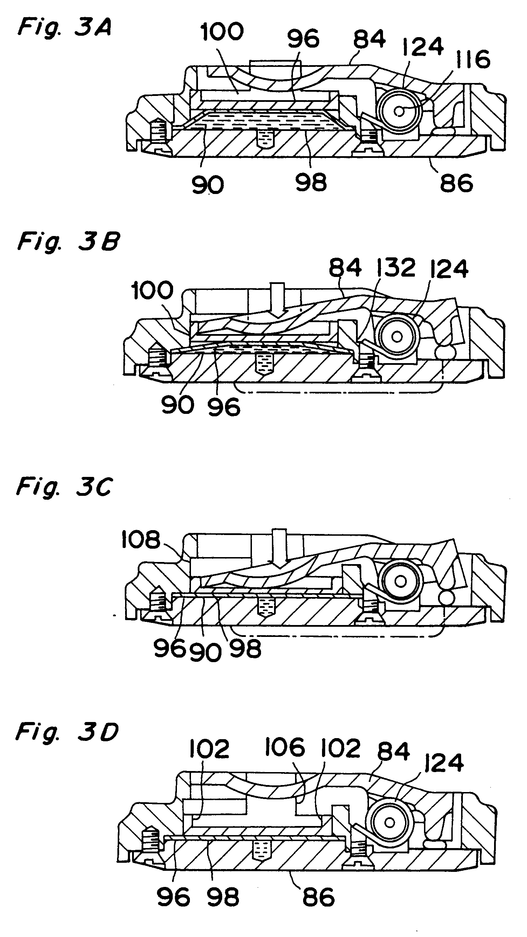

The above patient-controlled analgesic delivery device has, as shown in Fig. 3, a chamber 90 defined by a raised plateau 98 of a back plate 86 and a flexible circular sheet 96. This chamber 90 is connected to a first conduit communicating with a medical container and a second conduit communicating with a catheter or the like. Arranged on the flexible sheet 96 is a floating plate 100 which limits the volume of the chamber 100. Above the floating plate 100, there is arranged a push button 84 which allows the patient to operate the device by himself or herself. The base portion of the push button 84 is pivoted at its base portion by a pin 116 and biased by a coil spring 124 mounted around the pin 116. When the patient has pushed the push button 84, the chamber 90 is depressed by the floating plate 100, as illustrated in Figs. 3b and 3c, thereby delivering the liquid drug to the body of the patient through the conduit. When removing the finger from the push button 84, the button 84 is returned to its original state by the coil spring 124, as illustrated in Fig. 3d.

In the above device, however, the flexible sheet 84 is not restored to its original state by itself even when releasing the push button 84. The additional liquid drug can be introduced into the chamber 90 only by the pressure of the liquid drug squeezed out of the medical container which is so designed as to send out the liquid drug from a balloon by the restoring force of the balloon. However, it takes a long time to fill up the chamber 90 since the medical container is designed for microdose administration of the liquid drug and thus has a small volume of content. Accordingly, it is difficult to administer a additional dose of the liquid drug.

In addition, the above device is limited in dosage to the maximum volume of the reservoir which can not be selected optionally, thus making it difficult to administer the dosage of drug required for the patient in the critical moment. Another problem of the above patient-controlled analgesic delivery device is that it is complex in structure and thus high in manufacturing cost. Further, there is a fear of leakage of the liquid drug from the circumference of the flexible sheet 96.

It is therefore an object of the present invention to provide a self-administration device which is short in time for filling up, optionally selectable in dosage, free from any trouble such as leakage of liquids, and low in manufacturing cost.

The above and other object of the present invention are achieved by providing a self-administration device including:
a casing composed of a cylindrical container having an open end and a closed end at the opposite end, said closed end being provided with inlet and outlet ports for a liquid drag;
a reservoir arranged in said casing and mounted on the closed end of said casing, said reservoir being easily deformable by pressing and capable of being restored to its original shape; and
a pushing means for depressing said reservoir, said pushing means being movably arranged in or mounted on the open end of said casing so as to be moved along an inner or outer wall of said casing to press said reservoir; said inlet and outlet ports of said casing being opened to the interior of said reservoir.

In the preferred embodiment, a flow path including the outlet port of the casing is provided with a check valve to prevent the liquid drug from reversed flow. Preferably, the pushing means is provided at the inner bottom wall thereof with a projection to press the reservoir. Further, the reservoir is preferably made of an elastic material. The flow path including the inlet port for liquid drug may include a flow control means through which the reservoir can be connected to a medical container having the ability to deliver a liquid drug contained therein.

In use, when the patient has pushed the pushing means, the pushing means is moved along the inner wall or outer wall of the casing so that the reservoir is pressed by the projection formed on the inner bottom wall of the pushing means. Thus, the liquid drug contained in the reservoir is pressed out and delivered to the patient through the outlet port. After releasing the pushing means, the reservoir is restored by the restoring force thereof to its original state, causing occurrence of a negative pressure inside the reservoir. Thus, the liquid drug is absorbed into the reservoir.

Further scope of the present invention will become apparent from the detailed description given hereinafter with reference to the accompanying drawings. However, it should be understood that the detailed description and specific examples, while indicating preferred embodiment of the invention, are given by way of illustration only, since various changes and modifications within the spirit and scope of the invention will become apparent to those skilled in the art from this detailed description.
Fig. 1 is a cross sectional view of a self-administration device embodying the present invention;
Fig. 2 is a self-administration device of Fig. 1, with a medical container connected thereto through a flow control means; and
Fig. 3a - 3d is cross sectional views of an apparatus of the prior art, illustrating operation of the apparatus.

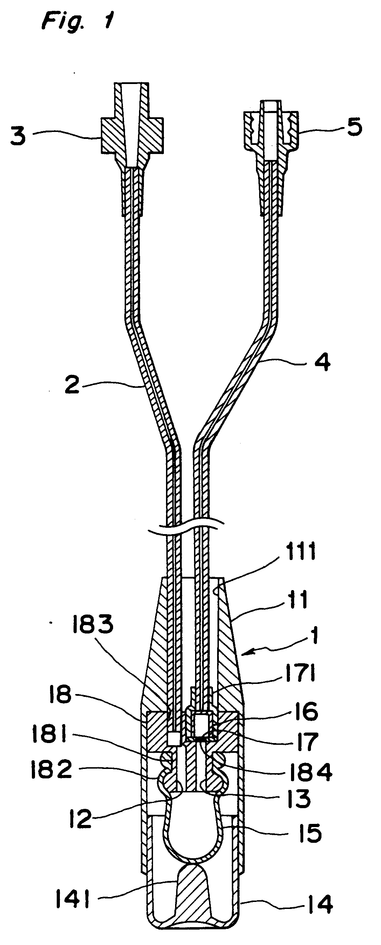

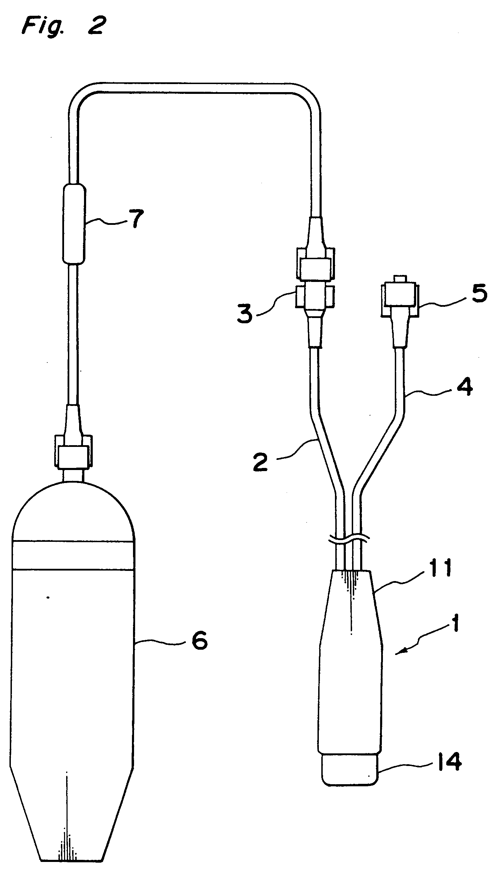

Referring now to Figs. 1 and 2, there is shown a self-administration device according to the present invention, which including a main body 1 comprising a cylindrical casing 11 provided at a closed end thereof with a drug inlet port 12 and a drug outlet port 13, a reservoir 15 housed in the casing 11, and pushing means 14 provided at an open end of the casing 11. The inlet port 12 and outlet port 13 are opened to the interior of the reservoir 15. The pushing means 14 is moved, by operating it with the finger, along an inner or outer wall of the casing 11, brought into contact with the reservoir 15, and then presses the reservoir 15 to deliver the liquid drug contained therein.

The casing 11 is a cylindrical member having an open end and an opposite closed end and is made of a transparent synthetic resin such as polyethylene, polypropylene, polyester or the like. The close end of the casing 11 is provided with a drug inlet port 12 and a drug outlet port 13. The closed end may be formed as an integral part of the casing 11. It is, however, preferred to constitute the closed end by a separate closing member 18, as shown in Fig. 1.

In the embodiment of Fig. 1, the casing 11 is formed into a cylindrical member with a stepped lumen and includes a closing member 18 mounted on the stepped portion of a large-sized lumen of the casing 11. The small-sized lumen of the casing 11 is used as an insertion hole 111 for attachment of tubes 2, 4. The closing member 18 is provided at one end with an outwardly extending projection 181 having a drug inlet port 12 and a drug outlet port 13. The use of such a closing member makes it easy to mount the reservoir 15 and a check valve 16 in the casing 11. The closing member 18 is so arranged in the large-sized lumen of the casing 11 so that the projection 181 is directed to the open end of the casing 11. The opposite side of the closing member 18 facing to the insertion hole 111 is provided with a hole 183 communicated with the drug inlet port 12 for connection with an upstream drug tube 2, and a hole 184 for connection with a downstream drug tube 4 or for insertion of a check valve 16, as illustrated in Fig. 1.

The projection 181 is provided with an annular rib 182 to prevent the reservoir 15 from being out of place. On the other hand, the closed end of the casing 11 may be provided with projections (not illustrated in the drawings) for connection with the upstream drug tube 2 and downstream drug tube 4 respectively, instead of provision of tube insertion hole 111.

The check valve 16 is used for prevention of back flow of the liquid drug from the downstream drug tube 4 toward the reservoir 15. Preferred check valves are of a duck bill type or of a ball valve type, though the check valve is not limited thereto. The check valve 16 is not necessarily provided in the device body 1, and may be arranged in the downstream drug tube 4 or in the Lure connecting means. In the preferred embodiment, the check valve 16 is arranged in a bore 184 of the closing member 18 and fixed thereto by a holding member 17 which has a connecting end 171 to which the downstream tube 4 is connected.

To the port portion 181 of the closing member 18 is attached a reservoir 15. Thus, the drug inlet port 12 and drug delivery port 13 open into the reservoir 15. The reservoir 15 is prevented by the annular rib 182 from slipping off. The reservoir 15 is a container reserving a liquid drug fed from the medical container 6 and is capable of being easily deformed by pressing (pressure deformable) and has the ability to be restored to its original shape when the pressure has been removed. To provide flexibility and restorability, the reservoir 15 is generally made of an elastic material such as flexible resin, natural rubbers, or synthetic rubbers. The preferred flexible resin includes polyethylene, polypropylene, polyester and the like, and the synthetic rubbers includes silicone rubber, olefine elastomers and the like.

The pushing means 14 is arranged in or on the open end of the casing 11 so as to be movable along the inner or outer wall of the casing 11. In the embodiment, the pushing means 14 is inserted in the open end of the casing 15 like as a pusher of a syringe so as to be moved along the inner wall of the casing 11 when pushed, while it may be mounted on the open end of the casing 15 so as to be able to move along the outer wall of the casing 11.The pushing means 14 is generally made of synthetic resin such as polyethylene, polypropylene or polyester in the form of a bottom-closed cylindrical member as shown in Fig. 1. When the pushing means 14 is pushed into the casing 1, it comes into contact with the reservoir 15 and depresses it at the inner wall of its bottom to deliver the liquid drug contained therein. In the preferred embodiment, it is preferred to provide a projection 141 extending inward from the inner wall of its bottom to make it easy to depress the reservoir 15.

To complete the self-administration device, the upstream drug tube 2 and downstream drug tube 4 are connected to the main body 1. These tubes are generally provided with a connecting means (e.g., Lure connector 3, 5 as illustrated in Fig. 1) for connection to other devices. The self-administration device may have liquid drug container 6 connected thereto directly or through a flow control means 7, as illustrated in Fig. 2. As the liquid drug container 6 to be connected to the self-administration device of the present invention, are those including a balloon, such as disclosed in U.S. patent 5178610 based on Japanese examined publication Nos. 6-77604 and 6-83725. The self-administration device of the present invention may be used by directly connecting it the dropper of an infusion set.

As mentioned above, the self-administration device of the present invention is easy to operate, takes a short time for recharging the reservoir with the liquid drug, and makes it possible to control the dose of the liquid drug, and free from troubles such as leakage. Thus, it is suitable for administration of a liquid drug to a patient by one shot. Further, it is inexpensive, thus making it possible to reduce a charge to the patient.

## Claims

1. A self-administration device for liquid drugs, including:
a casing composed of a cylindrical container having an open end and a closed end at the opposite end, said closed end being provided with inlet and outlet ports for a liquid drag;
a reservoir arranged in said casing and mounted on the closed end of said casing, said reservoir being easily deformable by pressing and capable of being restored to its original shape; and
a pushing means for depressing said reservoir, said pushing means being movably arranged in or mounted on the open end of said casing so as to be moved along an inner or outer wall of said casing to press said reservoir; said inlet and outlet ports of said casing being opened to the interior of said reservoir,

2. The self-administration device according to claim 1 wherein a flow path including said outlet port is provided with a check valve.

3. The self-administration device according to claim 1 or 2 wherein said pushing means is provided at its inner bottom wall with a projection adapted to press said reservoir.

4. The self-administration device according to any of the preceding claims wherein said reservoir is of an elastic material.

5. The self-administration device according to any of the preceding claims wherein a flow path including said inlet port is connected to a flow control means arranged therein, said flow control means being connected to a liquid drug container having the ability to deliver a liquid drug contained therein.
